# EUROPEAN PATENT APPLICATION

(11) **EP 3 379 438 A1**
(43) Date of publication of application: **26.09.2018**
(21) Application number: 17162709.4
(22) Date of filing: 24.03.2017
(51) Int. Cl.: G06F 19/00, A61F 2/30, A61F 2/28

(54) **CUSTOMIZED IMPLANT CREATION**

(71) Applicant: Koninklijke Philips N.V., 5656 AE Eindhoven (NL)
(72) Inventor: VOGTMEIER, Gereon, 5656 AE Eindhoven (NL)
(74) Representative: de Haan, Poul Erik

(57) **Abstract**

The present invention relates to customized creation of implants. In order to provide implants with improved adaptation, a device (10) for customized implant data set creation is provided. The device comprises an input interface (12) and an output interface (14); and a processing unit (16). The input interface is configured to provide a musculoskeletal system data set (18) of a patient to the processing unit into which an implant is to be inserted. For manufacturing a customized implant, the processing unit is configured to generate a customized implant data set (19) based on the musculoskeletal system data set.

## Description

### FIELD OF THE INVENTION

The present invention relates to a device for customized implant data set creation, to a system with such a device, to a method for creation of a customized implant data set, to a computer program element for controlling such a device and to a computer readable medium having stored the program element.

### BACKGROUND OF THE INVENTION

Implants are used, for example, in order to replace body structures, such as bones or joints. The implants may be provided to be adapted to the geometric conditions of the patient. As an example, it may be an intention to adapt the implant as best as possible to the patient considering other criteria than the geometric properties of the patient. WO 2013 134584 A1 describes a method of designing, generating, and fabricating custom implants by obtaining a 3D image of a site to receive an implant, simulating volumetric changes of the site, generating a virtual 3D implant, and fabricating a real 3D implant. However, it has been shown that implants should have an improved capability of being adapted.

### SUMMARY OF THE INVENTION

There may thus be a need to provide implants with improved adaptation.

The object of the present invention is solved by the subject-matter of the independent claims; further embodiments are incorporated in the dependent claims. It should be noted that the following described aspects of the invention apply also for the device for customized implant data set creation, for the system with a such device, for a method for creation of a customized implant data set, and for the computer program element for controlling such a device and also for the computer readable medium having stored the program element.

According to a first aspect of the present invention, a device is provided for customized implant data set creation comprising an input interface, an output interface and a processing unit. The input interface is configured to provide a musculoskeletal system data set of a patient to the processing unit into which an implant is to be inserted. The processing unit is configured to generate a customized implant data set based on the musculoskeletal system data set for manufacturing a customized implant.

The customized implant data set is created to get the best matching implant for the patient depending on the location of insertion in the body. By using the benefits of the enhanced data, e.g. from a spectral scanner or other suitable data acquisition systems, the implant can be constructed in an optimum way. Besides the geometry information, the data acquisition with an apparatus like the spectral scanner provides the additional information on the properties of the bone that are used to adapt the customized implant to the bone structure. This results in having the best matching combination between the bone structure and the implant.

The musculoskeletal system can also be referred to as human locomotive system, movement apparatus, locomotor apparatus or bone structure.

According to an example, the musculoskeletal system data set is generated by different imaging modalities comprising at least one of the group of a spectral computer tomography (CT) scanner, a spectral X-ray scanner, and a fusion of Magnetic Resonance Tomography (MRT) and a Computer Tomography (CT) scanner. By this, information about the soft tissue structure surrounding the bone can be obtained- e.g. muscles, tendons and / or fascias. This provides information about the dynamic forces at the location of the human body, into which e.g. an implant is to be inserted. As a result, the overlay of the soft tissue structure surrounding the bone information with the bone density and the geometry, provides the functional information of the musculoskeletal system. This information is used to customize the implant to the specific condition of the musculoskeletal system of a patient.

A CT scanner may include an X-ray tube that emits ionizing radiation that traverses an examination region and a portion of an object or subject therein and illuminates a detector array disposed across the examination region, opposite the X-ray tube. The detector produces projection data indicative of the detected radiation. The data can be reconstructed to generate volumetric image data indicative of the portion and the geometry of the object or subject. With spectral CT for example, the spectral data set or projection data includes signals which correspond to different photon energy ranges. In an example, the signals can be obtained concurrently by at least one detector. In another example, two energy signals can be obtained at the same time by two-layer detectors, e.g., a photon counting detector, by sequentially switching between at least two different peak kilovoltages (kVps). There are several approaches for performing spectral CT scanning. For example, the CT scanner may include two or more sources, and/or at least one source configured to switch between at least two different Peak Kilovoltages (kVps), and/or a detector array with energy-resolving detectors. In another example, the spectral data set can be obtained by applying different energy spectrum filtrations. With a simultaneous dual-layer detector (e.g. a Yttrium-based scintillator), the detector identifies photons of high energy and low energy simultaneously, allowing to not only view anatomy, but also view material content of critical structures. Therefore, the spectral data set is of value and provides the necessary information to adapt an implant in an optimum way to the detected material properties of the musculoskeletal system.

The musculoskeletal system data set can be generated by different imaging modalities like for example a fusion of an MRT system and a CT scanner to get enhanced imaging data of the bone structure, i.e. more material properties also for soft tissue (muscle) etc. as input data

According to an example, the processing unit is further configured to generate the customized implant data set also considering a patient specific load profile.

With this additional information of the patient specific load profile, it is further possible to adapt the implant to the patient specific properties. The patient specific load profile describes static and dynamic movement scenarios of the patient.

The patient specific load profile can list expected main activities of the natural movement process of the patient like sitting, standing, cycling, running, swimming, etc.

According to an example, the musculoskeletal system data set consists of bone geometry, bone density, bone health and/or tissue structure surrounding the bone.

The capturing of this information shows the value of the procedure with the spectral scan. In an example, it could also be foreseen to combine the spectral CT scanning with a standard DICOM (Digital imaging and communications in Medicine) CT geometry information.

The bone geometry signifies the dimension of the bone like length, height etc. in a three-dimensional coordinate system.

The bone density signifies the bone condition. The bone density can vary from patient to patient. This can contain information of the porosity of the bone structure for example.

The bone health signifies the bone state, i.e. if the bone is attrited or inflamed for example.

The tissue structure surrounding the bone comprises, for example, the muscle structure, the tendons structure, the connective tissue surrounding the bone, or fascia portions etc.

By considering the muscle structure, the implant can be adapted, for example, to the force transmission of the muscle to the bone, and the implant and the interface especially. This is different for each patient and could be taken into consideration.

According to an example, the customized implant data set comprises the size and shape of the implant, an interface shape of the implant to the bone structure, a position of fixation points on the implant and the bone structure, a method of the fixation; and/or use of materials.

The size and the shape of the implant contains information of the geometry of the implant. The interface shape of the implant to the bone structure contains also geometrical information at the interface point of the implant in order to best match to the interface point of the bone. The position of fixation points provides information of the arrangement of the fixation points on the implant. For example, position of fixation points relates to locations where screws or the like are arranged to fix the implant. The method of the fixation of the implant on the bone structure provides information, which fixation is used. It can be a screwed or glued connection, for example. Additionally, the data set can provide information on materials to be used in the implant. In an example, the implant is created with a single material or with multiple materials. For example, the implant with multi materials is provided to design the functional part of the implant and the interface part of the implant with biocompatible material (coating or porous printing to have the bone growing into the implant). Additional opportunities exist also in the design of the implant to change the material properties at the dedicated positions to adapt stiffness and/or flexibility for a homogeneous dynamic load matching design to reduce the risk of overloaded areas at the interface between implant and bone and/ or the fixture points.

In an example, the processing unit is configured to execute static simulations and dynamic simulations based on the musculoskeletal system data set and the patient specific load profile to generate the customized implant data.

In an example, the processing unit is configured to execute static simulations and dynamic simulations based on the musculoskeletal system data set and the patient specific load profile to generate the customized implant data by modifying a standard implant data set.

The term "standard implants" relates the use of standard fixtures and sizes. Using standard implants may result in that the surgeon can prepare the bone always in the same way, which, however, may have disadvantages, but at least the surgeon is used to the procedure. The combination of the advantages of both, i.e. standard implants and customized implants, could be combined by modifying a standard implant data set.

The standard implants can be listed in a catalogue listing standard implants based on destination of the implant in the body of the patient, size, etc.

The simulations could be automatically running in an iterative process of adapting the geometries starting from some standard values but considering the patient specific load profile and the musculoskeletal system data set.

The usage of the complete geometry information from the spectral dataset to make fully customized implants exactly fitting to the geometry of the (remaining) bone structure, has the advantage to adapt to the individual geometry of the patient.

It is also provided in one example to start the simulation from zero, i.e. no standard implant is selected and the customized implant data set is generated based on the musculoskeletal system data set and the patient load profile without starting from a standard implant.

By simulating the different combinations of the musculoskeletal system data set and the customized implant data based on the individual properties of the bone structure and the patient specific load profiles, it is possible to generate a perfect matching combination which is generated in the customized implant data set. For example, the interface between the implant and the bone with fixture points can be configured in such a way that forces that will be applied during the natural movement processes of the patient on the interface of bone structure and customized implant are adapted in a best manner to the properties of the patient.

According to an example, the processing unit is configured to execute static and dynamic simulations relating to patient specific load profile with a mechanical finite element analysis. The finite element analysis can simulate for example the forces interacting at the interface and at the individual areas of the implant to design an implant with best matching properties during dynamic interaction between bone structure and implant based on the patient load profile.

According to a second aspect of the invention, also a system is provided for creation of a customized implant data set. The system comprises a spectral scanner and the device for customized implant data set creation. The spectral scanner is configured to acquire spectral data of a patient and to generate a musculoskeletal system data set and to provide the musculoskeletal system data set to the device.

The spectral scanner can be a spectral computer tomography (CT) scanner, a spectral X-ray scanner, or a fusion of Magnetic Resonance Tomography (MRT) and a Computer Tomography (CT) scanner.

According to an example, the system further comprises a 3D-printer (three-dimensional printer). An output interface of the device is configured to transmit a generated customized implant data set to the 3D-printer. The 3D-printer is configured to manufacture an implant based on the customized implant data set.

According to an example, the system further comprises an image data set providing treatment guidance based on the customized implant data set.

The term "image data set" relates to image information of a virtual bone and customized implant. Based on this image data set, treatment guidance is provided to the surgeon, i.e. the surgeon can prepare the medical intervention. The virtual image data set can be provided in 2D or 3D data.

According to a third aspect of the invention, also a method is provided for creation of a customized implant data set. The method comprises the following steps.
a) Providing a musculoskeletal system data set; the musculoskeletal system data set is a spectral CT data set of a bone structure into which an implant is to be inserted; and
b) Generating a customized implant data set based on the musculoskeletal system data set.

According to an example, the method further comprises the step of providing a patient specific load profile, and in step b) a customized implant data set is generated also considering the patient specific load profile.

The step providing a patient specific load profile can be operated after providing a musculoskeletal system data set but can also be operated simultaneously.

The same applies for step b), i.e. the customized implant data set is generated considering the musculoskeletal system data set alone or by considering also the patient load profile.

According to another example, the method further comprises the steps of executing static simulations and dynamic simulations based on the musculoskeletal system data set and the patient specific load profile to generate the customized implant data, or executing static simulations and dynamic simulations based on the musculoskeletal system data set and the patient specific load profile to generate the customized implant data by modifying a standard implant data set.

These steps should not be considered as additional steps following the steps a) and b). These steps are showing the way of working of the processing unit when generating the customized implant data set.

According to an example, the method comprises the steps of
c) transmitting the customized implant data set to a 3D-printer; and
d) printing the implant based on the customized implant data set.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a device for creation of customized implant data set;
Fig. 2 shows an example of a system with such a device;
Fig. 3 shows another example of the system;
Fig. 4 illustrates an example of a method for creation of a customized implant data set; and
Fig. 5 illustrates an example of a method for manufacturing of an implant based on a customized implant data set.

### DETAILED DESCRIPTION OF EMBODIMENTS

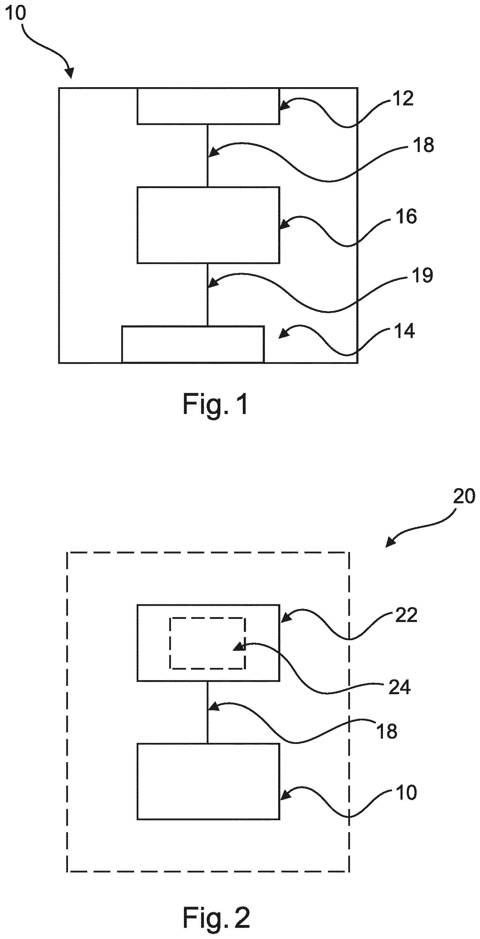

Fig. 1 shows a device 10 for customized implant data set creation. The device comprises an input interface 12 and an output interface 14 and a processing unit 16. The input interface is configured to provide a musculoskeletal system data set 18 of a patient to the processing unit, wherein the musculoskeletal system data set is a spectral data set of a bone structure into which an implant is to be inserted. The processing unit is configured to generate a customized implant data set 19 based on the musculoskeletal system data set for manufacturing a customized implant.

In an example, not further shown, the spectral data set is generated by different sources comprising a spectral computer tomography scanner, and/or a spectral X-ray; and/or a fusion of Magnetic Resonance Tomography and Computer Tomography scanner. In another example, also not shown, the processing unit is further configured to generate the customized implant data set also considering a patient specific load profile.

In a further example, also not shown, the musculoskeletal system data set comprises of data of a bone geometry, a bone density, bone health, and tissue structure surrounding the bone.

In a still further not shown example, the customized implant data set comprises data of size and shape of implant, interface shape of the implant to the bone structure, position of fixation points on the implant and the bone structure, a method of the fixation and/or the use of materials.

In another example, not shown in detail, the processing unit is configured to execute static and dynamic simulations relating to patient specific load profile with a mechanical finite element analysis.

Fig. 2 shows a system 20 for creation of a customized implant data set. The system 20 comprises a spectral scanner 22 and a device 10. The spectral scanner 22 is configured to acquire spectral data 24 of a patient and to generate a musculoskeletal system data set and to provide the musculoskeletal system data set 18 to the device. The spectral scanner can be a spectral CT scanner, a spectral X-ray scanner, and/or a MRT scanner.

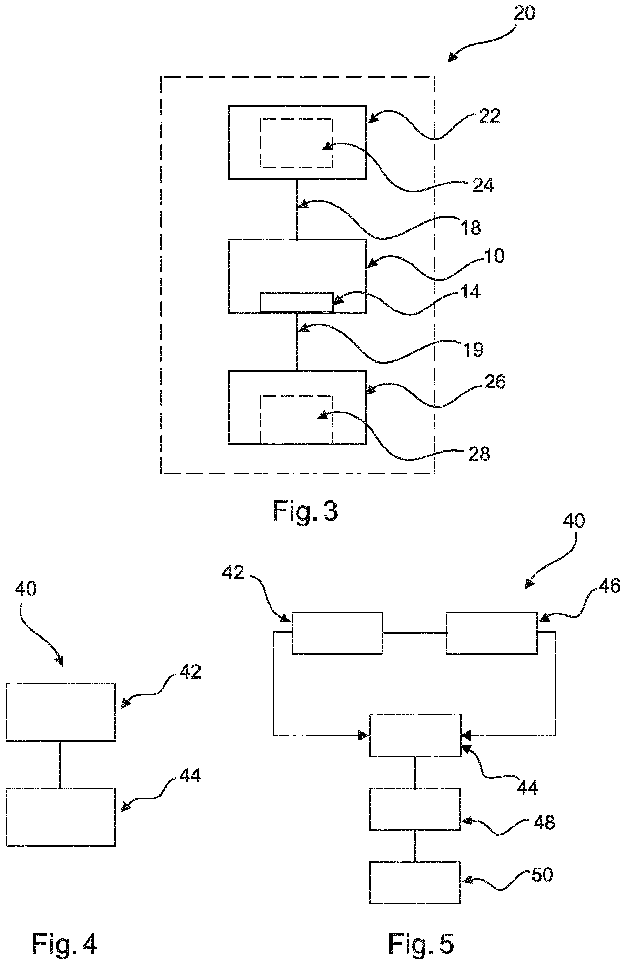

Fig. 3 shows another embodiment of the system 20. The system 20 further comprises a 3D-printer 26, wherein an output interface 14 of the device 10 is configured to transmit a customized implant data set 19 to the 3D-printer 26. This can be done directly or via a network in a hospital or to an external delivery service. The 3D-printer 26 is configured to manufacture an implant 28 based on the customized implant data set.

In an example, not further shown, the system further comprises an image data set providing treatment guidance based on the customized implant data set.

Fig. 4 shows basic steps of a method 40, wherein the method comprises the following steps: In a first step 42, also referred to as step a), a musculoskeletal system data set is provided. The musculoskeletal system data set is a spectral CT data set of a bone structure into which an implant is to be inserted. In a second step 44, also referred to as step b), a customized implant data set is generated based on the musculoskeletal system data set.

Fig. 5 shows another exemplary embodiment of the method 40. The method further comprises the steps of providing 46 a patient specific load profile. In step b), generating 44 a customized implant data set is also considering the patient specific load profile.

In an example, the customized implant data set is generated by the processing unit by executing static simulations and dynamic simulations iteratively (shown by the array) based on the musculoskeletal system data set and the patient specific load profile to generate the customized implant data.

In another example, the customized implant data set is generated by the processing unit by executing static simulations and dynamic simulations based on the musculoskeletal system data set and the patient specific load profile to generate the customized implant data by modifying a standard implant data set.

Further, once the customized implant data set is determined, in an example the method further comprises the steps of c) transmitting 48 the customized implant data set to a 3D-printer via a direct interface in a hospital or to an external delivery service, and d) printing 50 the implant based on the customized implant data set.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for customized implant data set creation, the device comprising:
- an input interface (12) and an output interface (14); and
- a processing unit (16);
wherein the input interface is configured to provide a musculoskeletal system data set of a patient to the processing unit into which an implant is to be inserted; and
wherein, for manufacturing a customized implant, the processing unit is configured to generate a customized implant data set (19) based on the musculoskeletal system data set.

2. Device according to claim 1, wherein the musculoskeletal system data set is generated by different imaging modalities comprising at least one of the group of:
- a spectral computer tomography scanner;
- a spectral X-ray; and
- a fusion of Magnetic Resonance Tomography and Computer Tomography scanner.

3. Device according to one of the preceding claims, wherein the processing unit is further configured to generate the customized implant data set also considering a patient specific load profile.

4. Device according to one of the preceding claims, wherein the musculoskeletal system data set consists of data of at least one of the group of:
- bone geometry;
- bone density;
- bone health; and
- tissue structure surrounding the bone comprising information of muscles, tendons and fascias.

5. Device according to one of the preceding claims, wherein the customized implant data set comprises data of at least one of the group of:
- size and shape of implant;
- interface shape of the implant to the bone structure;
- position of fixation points on the implant and the bone structure;
- method of the fixation; and
- use of materials.

6. Device according to one of the preceding claims, wherein the processing unit is configured to execute static and dynamic simulations relating to patient specific load profile with a mechanical finite element analysis.

7. A system (20) for creation of a customized implant data set, the system comprising:
- a spectral scanner (22); and
- a device (10) according to one of the preceding claims;
wherein the spectral scanner is configured to acquire spectral data (24) of a patient and to generate a musculoskeletal system data set; and to provide the musculoskeletal system data set to the device.

8. System according to claim 7, wherein the system further comprises:
- a 3D-printer (26);
wherein an output interface (14) of the device (10) is configured to transmit a generated customized implant data set (19) to the 3D-printer; and
wherein the 3D-printer is configured to manufacture an implant (28) based on the customized implant data set.

9. System according to claim 7 or 8, wherein the system further comprises:
- an image data set providing treatment guidance based on the customized implant data set.

10. A method (40) for creation of a customized implant data set, the method comprising the following steps:
a) providing (42) a musculoskeletal system data set into which an implant is to be inserted; and
b) generating (44) a customized implant data set based on the musculoskeletal system data set.

11. Method according to claim 10, wherein the method further comprises the following steps:
a1) providing (46) a patient specific load profile; and
wherein in step b), generating (44) a customized implant data set is also considering the patient specific load profile.

12. Method according to claim 10 or 11, wherein the method further comprises:
- executing static simulations and dynamic simulations based on the musculoskeletal system data set and the patient specific load profile to generate the customized implant data; or
- executing static simulations and dynamic simulations based on the musculoskeletal system data set and the patient specific load profile to generate the customized implant data by modifying a standard implant data set.

13. Method according to one of claims 10 to 12, further comprising:
c) transmitting (48) the customized implant data set to a 3D-printer; and
d) printing (50) the implant based on the customized implant data set.

14. A computer program element for controlling a device according to one of claims 1 to 9, which, when being executed by a processing unit, is adapted to perform the method steps of one of the claims 10 to 13.

15. A computer readable medium having stored the program element of claim 14.
